# EUROPEAN PATENT APPLICATION

(11) **EP 1 097 716 A1**
(43) Date of publication of application: **09.05.2001**
(21) Application number: 99402733.2
(22) Date of filing: 03.11.1999
(51) Int. Cl.: A61K 38/06, A61P 3/04

(54) **Dietary anti-adiposity food supplements and drugs based on thyrotropin-releasing hormone (TRH)**

(71) Applicant: Chronolife Inc., 6826 Riva san Vitale (CH)
(72) Inventor: Pierpaoli, Walter, 6595 Montedato-Riazzino (IT)
(74) Representative: Gutmann, Ernest

(57) **Abstract**

A composition, particularly dietary supplement having anti-obesity effects. Its main active component consists of thyrotropin-releasing hormone (TRH).

## Description

The invention relates to the preparation of food supplements for man or animal essentially based on thyrotropin-releasing hormone (TRH), a tripeptide, i.e. (pyro)Glu-His-Pro NH2 or salts thereof suitable for food consumption (hereafter designated as "dietary salts"), such as a TRH tartrate. These food supplements, when associated with food or beverage not only allow for a prevention of a weight increase as a result of the intake of food, but even a decrease of weight when adsorbed by beings having accumulated fats.

It is well known that obesity is often connected with a derangement of neuroendocrine control resulting from an accumulation of fat. Overfed children get deranged at a prepubertal time when their brain is still developing.

TRH is known to of provide for the restoration of thymic and thyroid functions which normally undergo decay in the course of aging, for the reduction in body fluids, particularly blood serum, of elderly people, of the contents of lipid-related compounds, e.g. cholesterols, triglycerides and phospholipids, associated with arteriosclerosis or related diseases, particularly arterial and venous thromboses. It has been shown, particularly in international application PCT/EP 90/00209, that TRH treatment of aging mice (18 month-old mice) causes the levels of cholesterol, triglycerids and phospholipids to be brought back to values currently observed in young mice, e.g. three month-old mice.

The results which have been observed in old mice shielded another effect, but more effectively observed in younger mice or obese mice, i.e. a reduction of fat depots, more particularly those resulting from the above mentionned neuroendocrine derangements. Actually it has been found that TRH can dissolve said fat depots rapidly in younger mice or in obese mice models upon mobilizing them rapidly as can be evidenced by a rapid increase of high levels of triglycerides in their circulation prior to then being burnt by oxidation. These effects result from the spectacular results provided by TRH in brain-lesioned obese mice as this will be exemplified later herein. These prima facie "opposite models" (assays in old mice and assays in brain-lesioned obese mice particularly in the anterior hypothalamic area (AHA)) provide evidence of the fact that the "dissolution" of said fat depots is neither mediated by a TRH activity on the thyroid gland, nor the result of an induction of an activation of thyroid hormone production.

The invention thus relates to compositions, preferably food additives, or even foods supplemented with such food additives (it being understood that the expression "food" as used herein is intended to encompass beverages), whose active component consists of TRH or dietary (or pharmaceutically) acceptable salts thereof. As already mentioned above, these compositions are active in preventing fat depots of being formed as a result of food intake or even causing fat depots that have already accumulated to be reduced. Of particular interest is the fact that, apparently, TRH has no substantial appetite satiation effect.

Any dietary salt suitable for intake y man or animal can be used. Watersoluble salts are preferred. Tartrates have already mentionned as consisting of particularly valuable salts for fulfilling such purposes. Examples of other suitable salts are acetates, citrates, etc. Needless to say that when reference is made herein to TRH, it must be understood that these salts are also encompassed by that abbreviation, except when and if stated otherwise.

TRH or its salts, on the one hand, and food, on the other hand, need not of course be adsorbed simultaneously. They can be absorbed sequentially. TRH can be absorbed alone e.g. in the morning prior to any food intake or between successive meals.

When used as a food additive or supplement, TRH will of course be absorbed orally before, after, simultaneously with food intake or between meals. Intake of TRH can occur either in solid form or, particularly when in the form of a sufficiently soluble salt, with drinking water or other beverages. Advantageous daily doses may range between 1 and 20 mg, it being understood that such figures are in no way limitative of the amounts that can be taken. TRH is an absolutely safe molecule and far greater amounts thereof can be absorbed without any risk.

While TRH may indeed be associated with other compounds active as " oxidative burners" of fat depots. It is preferred that the associated compounds not exert a satiety effect of their own, e.g. like cholecystokinin or an active peptide fragment thereof, such as the C-terminal octapeptide known under the abbreviation CCK-8 or under the now classical designation "sincalide". Indeed preferred compositions aim at normalizing body weight, particularly in obese people or people having a tendency towards obesity (or obese animals or animals having a tendency towards obesity), more generally of upkeeping fat deposits or fats that normally form as a result of food intake under control even in persons with normal endocrine functions, yet without interfering with normal appetite of those who absorb them.

Thus preferred compositions of the invention are free of appetite-cutter or satiation-causing components. They may of course include other components capable of stimulating the endogenous production of TRH in the host, e.g. melatonin.

While TRH or its salts are preferably used as food dietary supplements, they are also suitable for use in the manufacture of drug compositions for control of adiposity, including the cure of obesity. The above dosages are indicative of the daily doses that can be used for therapeutical purposes by the oral route. TRH may be administered in the form of capsules containing 5mg each. Though these drug compositions are preferably in a form (solid or liquid) suitable for administration by the oral route, it goes without saying that other administration routes, e.g. any parenteral route, can be contemplated. Again like in the above-mentionned domain of use (food dietary supplements) TRH can be associated with other components which are likely to also stimulate the endogeneous production of TRH in the host, e.g. melatonin.

Whatever the use contemplated, preferred TRH compositions of the invention are in a form which will provide for a protection of the active principle against the gastric acidity. TRH is either the base or the salt, e.g. the tartrate. Preferred embodiments of the invention comprise TRH- containing capsules coated with acid-(stomach)-resistant pellicles, of any nature e.g. cellulose films suitable for providing a more uniform release in the guts rather than in the stomach.

The anti-obesity properties of TRH are hereafter further illustrated by significant pharmacological data (as compared to results obtained in controls) presented hereafter in the tables. Of course they are only provided for illustrative purposes and they should by no means be construed as likely to limit the scope of protection afforded by the claims which follow. The conditions under which the tests were carried out, as well as the results presented, especially in the captions of said tables are eloquent enough to render a further discussion thereof useless.

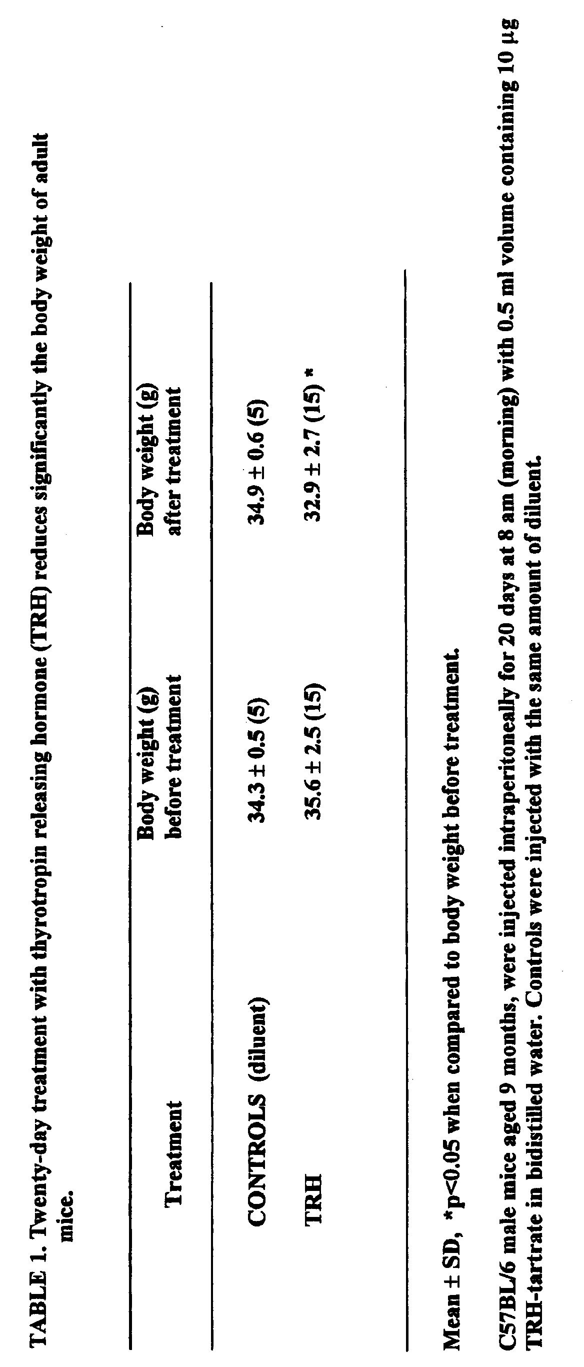

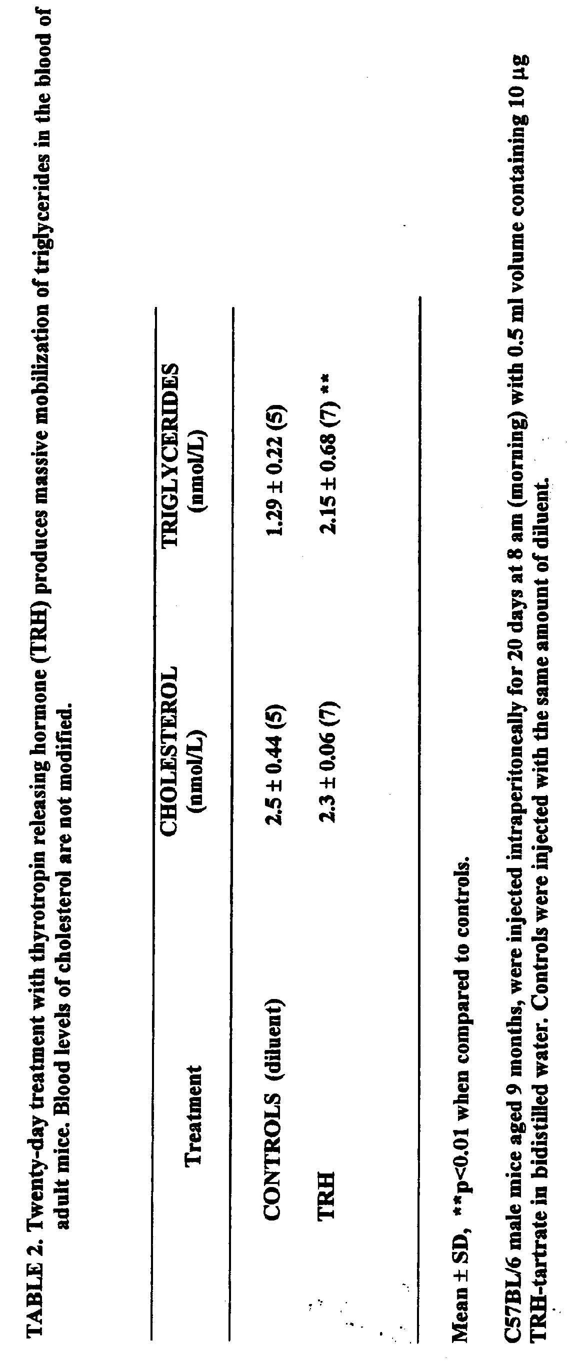

**TABLE 3.**

| Twenty-day treatment with thyrotropin releasing hormone (TRH) normalizes body weight of obese, anterior hypothalamic area (AHA)-lesioned mice. The mice reacquire the original obesity after interruption of TRH administration. | | |
|---|---|---|
| Body weight (g) | AHA-lesioned + TRH (N=6) | Sham-operated + diluent (N=6) |
| Before treatment | 39.0 ± 6.2 | 29.6 ± 2.9 |
| After 10 days of Treatment | 33.7 ± 4.2 | 27.7 ± 2.3 |
| After 20 days of Treatment | 32.0 ± 3.6^{∗} | 27.6 ± 1.8 |
| After withdrawal Period of 5 days | 31.1 ± 3.4^{∗} | 26.8 ± 2.3 |
| After withdrawal Period of 60 days | 38.3 ± 5.9 | 29.3 ±4.1 |
| Mean ± SD, ^{∗}p<0.05 when compared to body weight before treatment | | |

C57BL/6 male mice aged 7 months (AHA-lesioned at 2 months of age to induce obesity), were injected intraperitoneally for 20 days at 6 pm (evening) with 0.5 ml volume containing 10 µg TRH-tartrate in bidistilled water. Controls were injected with the same amount of diluent.

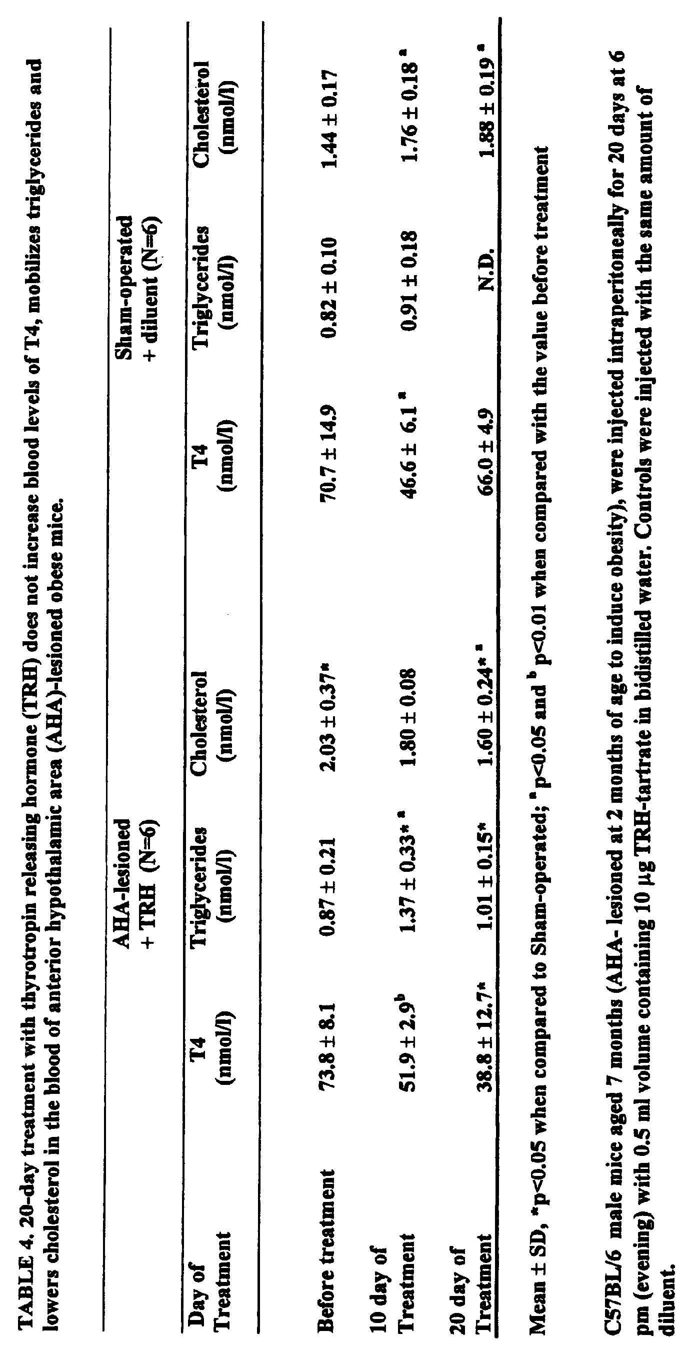

**TABLE 5.**

| 48-day treatment with thyrotropin releasing hormone (TRH) normalizes the body weight of anterior hypothalamic area (AHA)-lesioned obese mice. | | |
|---|---|---|
| Body weight (g) | AHA-lesioned + TRH (N=4) | AHA-lesioned + diluent (N=5) |
| Before treatment | 42.2 ± 6.4 | 38.1 ± 3.2 |
| After 27 days of Treatment | 39.2 ± 7.2 | 37.6 ± 2.4 |
| After 48 days of Treatment | 32.6 ± 4.4^{∗} | 36.7 ± 2.9 |
| Mean ± SD, ^{∗}p<0.05 when compared to body weight before treatment | | |

C57BL/6 female mice aged 8 months (AHA-lesioned at 2 months of age to induce obesity), were injected intraperitoneally for 48 days at 6 pm (evening) with 0.5 ml volume containing 10 µg TRH-tartrate in bidistilled water. Controls were injected with the same amount of diluent.

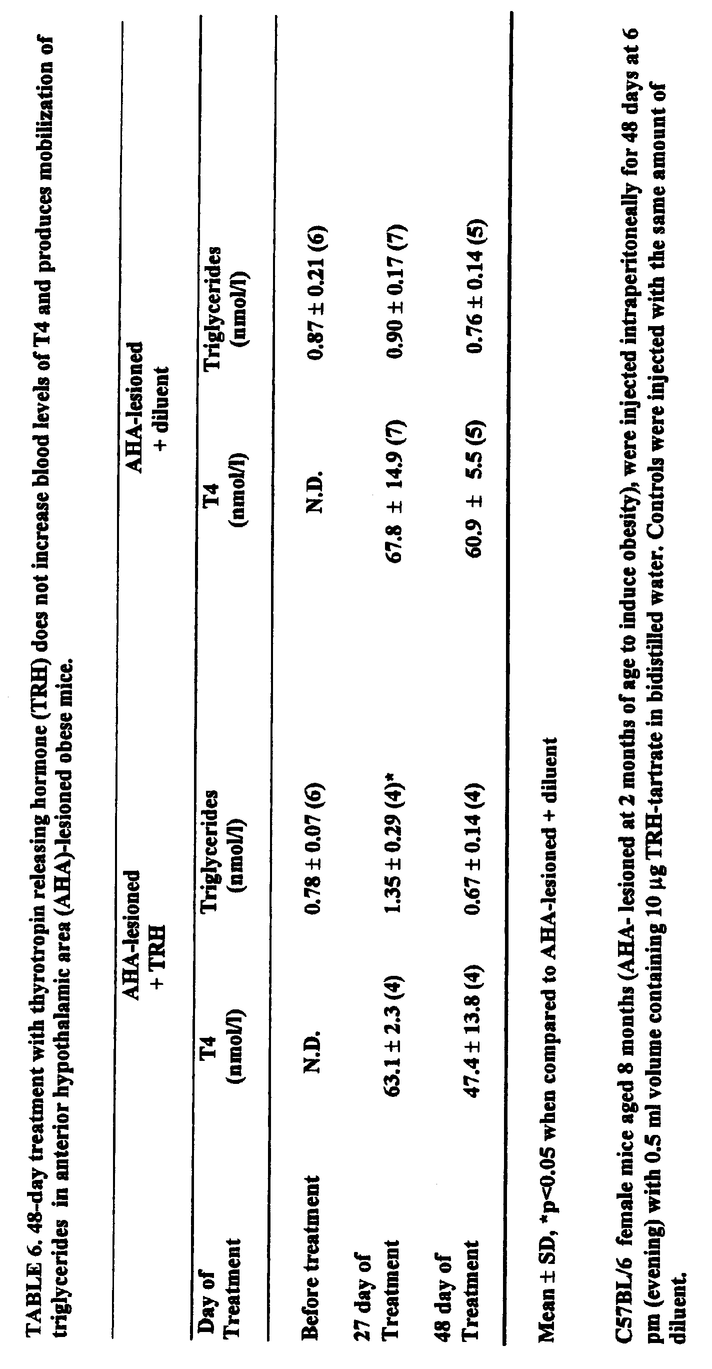

Increasing amounts of TRH do not modify thyroid hormone levels, thus demonstrating that TRH cannot increase, beyond a certain level, synthesis and release of thyroid hormones even at those very high dosages of parenteral administration:

This is shown by Table 7 hereafter which report the results of assays which were ran under the following conditions.

C57BL/6 male mice aged 9 months, were injected intraperitonealy for 20 days at 6 p.m. (evening) or at 8 a.m. (morning) with 0.5 ml volumes containing either 10 µg or 100 µg of TRH in bidistilled water. Controls were injected with the same amount of diluent. The mice were bled from the retroorbital venus plexus under rapid acute ether anaesthesia at 10 a.m. T3, T4 and TSH were measured by radioimmunoassay in the serum of individual mice.

**TABLE 7:**

| **Effect of 20 days treatment with thyrotropin releasing hormone (TRH) on blood levels of T3, T4 and TSH in mice.** | | | | | |
|---|---|---|---|---|---|
| Groups (G) | Treatment | No. | T3 (nmol/L) | T4 (nmol/L) | TSH (µU/mL) |
| 1 | CONTROLS (diluent) | 4 | 0.76 ± 0.06 | 47.2 ± 8.1 | 0.011 ± 0.005 |
| 2 | TRH morning 100 µg | 4 | 0.84 ± 0.02 | 74.9 ± 13.3 | 0.007 ± 0.005 |
| 3 | TRH evening 100 µg | 3 | 0.91 ± 0.02 | 68.7 ± 13.1 | 0.004 ± 0.005 |
| 4 | TRH morning 10 µg | 4 | 1.03 ± 0.07 | 82.0 ± 1.9 | 0.009 ± 0.006 |
| 5 | TRH evening 10 µg | 4 | 0.82 ± 0.08 | 65.7 ± 4.2 | 0.005 ± 0.004 |
| Mean ± SD, T3: G2 versus G4 = p<0.01; G3 versus G5 = NS | | | | | |
| T4: G2 versus G4 = NS; G3 versus G5 = NS | | | | | |
| TSH: G2 versus G4 = NS; G3 versus G5 = NS | | | | | |

The results of Table 7 demonstrate that chronical administration of high amounts of TRH cannot induce a state of permanent hyperthyroidism, with all its toxic and dangerous cardiovascular effects. This also demonstrates that the effects of TRH are only minimally mediated via its activity on hypophyseal TSH (Thyroid stimulating hormone). It also shows the lack of acute toxicity of TRH injected at huge dosages. The same in true with increasing doses of melatonin in the drinking water, which are not toxic.

The very basic Table 8 shows that oral, chronic administration of TRH in the drinking water of aging mice is effective in preventing the typical increase of body weight and adiposity which accompanies the initial stage of aging, which is also visible in humans everywhere! With other words, progressive alteration of pineal-hypothalamic-pituitary-peripheral endocrine glands function in the course of aging leads to the most typical appearance of decreased tolerance to glucose (hyperglycemia), increased levels of lipids, in particular triglycerides and cholesterol (hyperlipidemia) and progressive accumulation of fat, which is not obesity but the most common expression of hormonal derangements. TRH taken chronically will maintain normal body weight in normal aging mice, while the normal controls put on fat and weight.

The results reported in Table 8 clearly support the presumption that TRH is also capable of preventing onset of aging-related adiposity in humans.

**TABLE 8 :**

| **TRH given chronically by oral route prevents the increase of aging-related adiposity in old female mice.** | | |
|---|---|---|
| Treatment | Body weight (g) before treatment | Body weight (g) after treatment |
| Old controls | 33.5 ± 4.6 (13) | 37.2 ± 3.6 (13)^{∗} |
| Old + TRH | 33.1 ± 3.2 (15) | 33.8 ± 4.0 (15) |
| Mean ± SD, ∗p<0.05 when compared to body weight before treatment. | | |
| Treatment with TRH-tartrate in the drinking water (100µg/ml) was started in 16 month-old BALB/cJ X C57BL/6 F1 female mice, and continued for 5 months. | | |

Evidence that the lipid related compounds whose proportions in the blood circulation of old mice have a cause different from those observed in young mice treated with TRH seems further indicated by the fact that the weight of 16 month-old female mice, thus older mice which tend to accumulate age-related adiposity, can also be controlled and even reduced by the adsorption of TRH (see table 8). Indeed the contents in lipid-related compounds in the blood circulation of older mice (20 month-old mice) treated with TRH remain substantially unaltered, as evidenced by the results shown in Table 9 hereafter. The dosages of TRH were the same as in the assay reported in relation to Table 7.

**TABLE 9 :**

| **Effect of 2 months treatment with TRH on blood levels of cholesterol and triglycerides in old mice.** | | | |
|---|---|---|---|
| Parameters measured | Young∗∗∗ (n = 8) | Old (n = 10) | Old + TRH (n = 10) |
| CHOLESTEROL (mmol/l) | 1.47 ± 0.21 | 1.81 ± 0.71 | 1.65 ± 0.29 |
| TRIGLYCERIDES (mmol/l) | 0.96 ± 0.18 | 1.17 ± 0.29 | 1.19 ± 0.46 |
| Mean + SD, | | | |
| Treatment with TRH was started in 20-month-old BALB/cJ female mice ∗∗∗ 3 - 4 month-old mice | | | |

It has also been found that the activities of melatonin and TRH can potentiate each other considerably when they are administered simultaneously or sequentially, it being nevertheless understood that in the latter instance the time interval which would separate the two administrations should nevertheless be small enough to provide for the simultaneous presence of melatonin and TRH in the blood circulation of the host. Particularly, the invention relates to pharmaceutical compositions containing both thyrotropin-releasing hormone and melatonin in relative amounts providing for a synergistic effect of their respective anti-obesity activities.

Further preferred compositions of the invention comprise both TRH and melatonin. They provide for enhanced metabolizing effect of fat depots.

Illustrative of the proportions of the two components which can be used in suitable pharmaceutical compositions are the following relative weight ratios:
- of from 1 to 20 mg of thyrotropin-releasing hormone to of from 0.1 to 50 mg of melatonin, or
- of from 5 to 10 mg of thyrotropin-releasing hormone to of from 0.5 to 25 mg of melatonin.

Thus the invention relates more particularly to compositions for systemic administration to man or animal (or to the preparation of such compositions, particularly for the treatment of obesity of both melatonin and TRH in relative synergistically effective amounts, in association with a suitably physiologically acceptable carrier chosen depending upon the required route of administration. The invention relates also more particularly to compositions containing both melatonin and TRH associated with ointments, lotions or any other vehicles allowing for topical applications of said compositions.

Preferred conditions of use of TRH and melatonin, as associated with each other, with a view of achieving the above-mentioned results comprise administering to the living host the relative effective doses of said TRH and melatonin in one of the administration forms which have been mentioned above, preferably from sunset, or a little earlier, say from 4 P.M., up to the time where the host goes to sleep. Examples of said daily doses capable of inducing a favorable effect, range from 0.1 to 1 mg of TRH and of from 0.01 to 1 mg of melatonin per kg of body weight, when the administration is effected by the oral or rectal route, or 0.005 to 0.1 mg of TRH and of from 0.01 to 1 mg of melatonin, per kg of body weight, when administered by the parenteral route. For purpose of illustration only, topical compositions may contain from 0.005 to 5% in weight of melatonin and from 0.001 to 5% in weight of TRH. TRH and melatonin may also be administered by other routes, e.g., by the rectal route.

## Claims

1. A process for the production of a composition for the control of fat depots, wherein the active principle of that composition consists of thyrotropin-releasing hormone (TRH) or of a salt thereof suitable for animal or human consumption.

2. The process of claim 1 for the production of a composition which is free of components causing the appetite of the persons or animals which absorb it to be reduced.

3. The process of claims 1 or 2 which also contains melatonin or a salt thereof suitable for human or animal consumption.

4. The process of any of claims 1 to 3 which is a dietary supplement for food or beverages.

5. A composition which contains an amount of TRH or of a salt thereof suitable for consumption effective to induce in a person or animal which absorb them, a decrease of the fat deposits.

6. A composition according to claim 5 which are free of components causing the appetite of the person or animal which absorbs them to be reduced.

7. A composition according to claim 5 or 6 which also contains melatonin or a salt thereof which is suitable or a salt thereof which is suitable for human or animal consumption.

8. The composition of any of claims 5 to 7 which is a food or beverage.

9. The composition of any of claims 5 to 7 which is a drug composition having anti-obesity properties.

10. The composition of any of claims 5 to 9 which contains weight ratios of from 1 to 20 mg of TRH and from 0.1 to 50 mg of melatonin.

11. The composition of claim10 which contains weight ratios of from 5 to 10 mg of TRH and from 0.5 to 25 mg of melatonin.
